(19) Europäisches Patentamt
European Patent Office
Office européen des brevets

(11) Publication number: 0 267 622 A2

(12) **EUROPEAN PATENT APPLICATION**

(21) Application number: 87116787.0

(22) Date of filing: 13.11.87

(51) Int. Cl.4: G01N 33/569 , G01N 33/552 , //C12N15/00,C12N5/00,C12P21-/00

The microorganisms have been deposited with the Fermentation Research Institute under number FERM BP - 863 and with the American Type Culture collection under number 53228.

The applicant has filed a statement in accordance with Rule 28 (4) EPC (issue of a sample only to an expert). Accession number(s) of the deposit(s): FERM BP - 863 and ATCC 53228.

(30) Priority: 14.11.86 JP 271606/86

(43) Date of publication of application: 18.05.88 Bulletin 88/20

(84) Designated Contracting States: DE FR GB IT

(71) Applicant: KYOWA HAKKO KOGYO CO., LTD.
6-1, Ohte-Machi Itchome
Chiyoda-ku Tokyo(JP)

(72) Inventor: Fukui, Masanori
1-12-7, Nozawa
Setagaya-ku Tokyo(JP)
Inventor: Kuga, Tetsuro
2-3-107, Kyowa-machi
Hofu-shi Yamaguchi-ken(JP)
Inventor: Yamasaki, Motoo
3-6-6, Asahi-machi
Machida-shi Tokyo(JP)
Inventor: Itoh, Seiga
218-14, Aza Hachimannishi Aihara
Sagamihara-shi Kanagawa-ken(JP)

(74) Representative: Vossius & Partner
Siebertstrasse 4 P.O. Box 86 07 67
D-8000 München 86(DE)

(54) **Method for detecting anti-adult T cell leukemia virus antibody.**

(57) The present invention provides a method for detecting an anti-adult T cell leukemia virus antibody in a sample according to an immunological procedure using a polypeptide encoded by a fused gene comprising the gag gene of ATLV or a part thereof and the env gene of ATLV or a part thereof as an antigenic substance.

EP 0 267 622 A2

## METHOD FOR DETECTING ANTI-ADULT T CELL LEUKEMIA VIRUS ANTIBODY

So far, antigen-antibody reaction has been used in various fields, specifically in the field of medical treatment such as diagnosis, therapy and prevention of diseases. The usefulness of antigen-antibody reaction is due to its high specificity which assures correctness of diagnosis and effectiveness of therapy and prevention of diseases.

Adult T cell leukemia (hereinafter referred to as ATL) has been presumed to be caused by adult T cell leukemia virus (hereinafter referred to as ATLV; a synonym of human T cell leukemia virus which is hereinafter referred to as HTLV-1), and serum diagnosis of ATLV (HTLV-1) infection is important for the diagnosis of ATL, the prevention of ATLV infection, etc. Diagnosis of ATLV infection is carried out by detecting the presence of an antibody to an ATL-associated antigen (hereinafter referred to as ATLA) in serum through antigen-antibody reaction. Detection of an anti-ATLV antibody have so far been conducted by using, as an antigenic substance, p24 which is a product of the gag gene of ATLV (the method using p24 is hereinafter referred to as p24 method), a polypeptide which is a gene product of the whole ATLV, or an env gene product.

In the above-described detection of an anti-ATLV antibody by known methods, diagnosis results are sometimes found to be false positive or false negative, and development of a more reliable detection method has been desired.

The present invention relates to a method for detecting an anti-ATLV antibody in a sample according to an immunological procedure using a polypeptide encoded by a fused gene comprising the gag gene of ATLV or a part thereof and the env gene of ATLV or a part thereof as an antigenic substance. Thus, the present invention is useful in the field of clinical diagnosis.

According to the present invention, an anti-ATLV antibody can be detected with high precision and serum diagnosis of ATL can be effectively carried out. Furthermore, the method of the present invention is applicable to diseases showing a cross reaction with p24.

Fig. 1 shows the steps for constructing plasmid pAFB7.

Fig. 2 shows the steps for constructing plasmid pAHA1.

Fig. 3 shows the steps for constructing plasmid pAFG10.

Fig. 4 shows the steps for constructing plasmid pETI7.

Fig. 5 shows a correlation diagram on which the antibody value of the Eisai method is plotted on the Y-axis and the antibody value of the p24 method is plotted on the X-axis. Correlation coefficient is 0.8924. According to the method of least squares (primary regression) by simple frequency distribution, the regression equation will be $Y = 2.7437X + 0.2151$.

Fig. 6 shows a correlation diagram on which the antibody value of the Eisai method is plotted on the Y-axis and the antibody value of the gag-env method is plotted on the X-axis. Correlation coefficient is 0.7479. According to the method of least squares (primary regression) by simple frequency distribution, the regression equation will be $Y = 1.9473X - 0.1119$.

The present invention relates to a method for detecting an anti-ATLV antibody, characterized by detecting the anti-ATLV antibody in a sample according to an immunological procedure using a polypeptide encoded by a fused gene comprising the gag gene of ATLV or a part thereof and the env gene of ATLV or a part thereof as an antigenic substance.

Any polypeptide can be used as an antigenic substance in the present invention, so far as it is encoded by a fused gene comprising the gag gene or a part thereof and the env gene or a part thereof. Preferably, a polypeptide having the following amino acid sequence which is prepared according to the procedure shown in Reference Example 1 (hereinafter referred to as gag-env protein) is used. The gag-env protein is a polypeptide which comprises a polypeptide having the sequence from the 14th amino acid (proline) to the 139th amino acid (glycine) of the N-terminal region of p24 and a polypeptide having the sequence from the 197th amino acid (threonine) to the 295th amino acid (proline) of the N-terminal of env, with additional methionine-aspartic acid attached to the N-terminal and lysine (Lys) attached to the C-terminal.

Met Asp Pro Trp Gln Met Lys Asp Leu Gln Ala Ile Lys Gln Glu
Val Ser Gln Ala Ala Pro Glu Ser Pro Gln Phe Met Gln Thr Ile
Arg Leu Ala Val Gln Gln Phe Asp Pro Thr Ala Lys Asp Leu Gln
Asp Leu Leu Gln Thr Leu Cys Ser Ser Leu Val Ala Ser Leu His
His Gln Gln Leu Asp Ser Leu Ile Ser Glu Ala Glu Thr Arg Gly
Ile Thr Gly Tyr Asn Pro Leu Ala Gly Pro Leu Arg Val Gln Ala
Asn Asn Pro Gln Gln Gln Gly Leu Arg Arg Glu Tyr Gln Gln Leu
Trp Leu Ala Ala Phe Ala Ala Leu Pro Gly Ser Ala Lys Asp Pro
Ser Trp Ala Ser Ile Leu Gln Gly Thr Leu Gln Ser Thr Asn Tyr
Thr Cys Ile Val Cys Ile Asp Arg Ala Ser Leu Ser Thr Trp His
Val Leu Tyr Ser Pro Asn Val Ser Val Pro Ser Ser Ser Ser Thr
Pro Leu Leu Tyr Pro Ser Leu Ala Leu Pro Ala Pro His Leu Thr
Leu Pro Phe Asn Trp Thr His Cys Phe Asp Pro Gln Ile Gln Ala
Ile Val Ser Ser Pro Cys His Asn Ser Leu Ile Leu Pro Pro Phe
Ser Leu Ser Pro Val Pro Thr Leu Gly Ser Arg Ser Arg Arg Ala
Val Pro Lys

The present invention can be applied to samples such as body fluids (e.g. blood), urine, etc.

The following immunological procedures can be used in the present invention according to the disclosures in the literature shown below:

o Enzyme immunoassay (hereinafter referred to as EIA), Eiji Ishikawa, et al.: Enzyme Immunoassay Procedure, published by IGAKU SHOIN LTD. (1978).

o Radio immunoassay (hereinafter referred to as RIA), Maya Inai, et al.: Immunoserology, published by ISHIYAKU SHUPPAN PUBLISHERS, INC. (1981).

o Fluorescence immunoassay (hereinafter referred to as FIA), ditto.

o Reversed passive agglutination, ditto.

o Phytohematoagglutination, ditto.

o Laser nephrometry, ditto.

o Immune hemolysis (procedure using erythrocytes or liposome), ditto.

o Particle agglutination, Saishin kensa, Vol. 2, No. 2, 151-157 (1984), published by Iten-sha, and Gann 75, 845 (1984).

In RIA, EIA and FIA, not only a homogeneous assay, but also a heterogeneous assay such as a sandwich method, etc. can be used.

An example of EIA according to the sandwich method is given below:

A well in a microtiter plate is coated with an antigen substance, and a serum sample diluted with an appropriate buffer solution such as buffer solution A [a 1/15 M sodium phosphate buffer solution containing 0.15 M NaCl, 0.1% galatin, 1% bovine serum albumin and 0.1% $NaN_3$ (pH 7.2)] is added to the well. After being allowed to stand at 4-37°C for one hour to one week, the well is thoroughly washed with an appropriate buffer solution such as buffer solution B [a 1/15 M sodium phosphate buffer solution containing 0.05% Tween 20 and 0.15 M NaCl (pH 7.2)]. Then a conjugate prepared by combining a Fab fragment of rabbit anti-human immunoglobulin antibody with horseradish peroxidase by the periodic acid method is diluted with an appropriate buffer solution, for example, buffer solution B, and the resulting solution is added to the well. After being allowed to stand at 4-45°C for 1-24 hours, the well is washed with the same buffer solution as above. Then, an appropriate substrate solution, for example, an orthophenylenediamine substrate solution [a 1/15 M sodium phosphate buffer solution containing 0.02% orthophenylenediamine and 35 mM hydrogen peroxide (pH 7.2)] is added to the well and the plate is allowed to stand at 15-37°C for 5-60 minutes. The reaction is discontinued with 2 N $H_2SO_4$ and the plate is allowed to stand at room temperature for 1-10 minutes. Then, absorbancies (O.D.) at 410 nm and 600 nm are measured with a photometer for

microtiter plate, and an antibody value is obtained as a value left by subtracting the absorbancy at 600 nm from that at 410 nm.

The foregoing procedure is one example, and the antibody, the substrate solution, the coloring agent, etc. can be changed when desired.

The method of the present invention using the gag-env protein is a distinguished method for detecting an anti-ATLA antibody, and a similar distinguished effect can be expected by the use of a combination of a gag gene product and an env gene product fixed on a single carrier (hereinafter referred to as a cocktail antibody polypeptide). The cocktail antibody polypeptide can be prepared by physically or chemically adsorbing a gag gene product and an env gene product onto an appropriate carrier, for example, liposomes or a solid phase carrier made of synthetic resin or glass.

The method of the present invention has a distinguished effect on the detection of an anti-ATLV antibody, and is applicable to diagnosis and prevention of TSP (tropical spastic paraplesia), HAM (HTLV-1-associated myelopathy) and MS (multiple sclerosis) which are known as diseases showing a cross reaction with p24 and various cancers other than ATL (for example, hepatoma and uterine carcinoma).

Certain specific embodiments of the invention are illustrated by the following representative examples.

## Example 1

Detection of anti-ATLV antibodies in serums of ATL patients and HTLV-1 carriers and normal human serum was carried out according to EIA using the gag -env protein as an antigen substance (gag-env method) or using p24, a fragment of a gag gene product (p24 method) and Eisai method using natural ATLV (Eitest® ATL, a product of Eisai Co., Ltd., Japan).

(1) EIA using the gag -env protein and p24 (sandwich method):

As serum samples, serums of ATL patients and HTLV-1 carriers (a total of 57 individuals) and 30 normal men were used. As the gag-env protein, a polypeptide obtained according to the procedure shown in Reference Example 1 was used, and as p24, a gag polypeptide obtained according to the procedure disclosed in Japanese Published Unexamined Patent Application No. 61534/85 was used.

Wells in microtiter plates (a product of Nunc Co.) were coated with 1 µg of the gag-env protein and 3 ug of p24, respectively.

Each serum sample was diluted 20-fold with buffer solution A (a 1/15 M sodium phosphate buffer solution containing 0.15 M NaCl, 0.1% gelatin, 1% bovine serum albumin and 0.1% $NaN_3$, pH 7.2),-and 100 µℓ each of the diluted serum was placed in the gag-env protein-coated and p24-coated wells. After being allowed to stand at room temperature for 2 hours, the wells were washed three times with 150 µℓ each of buffer solution B [a 1/15 M sodium phosphate buffer solution containing 0.05% Tween 20 (a product of Wako Pure Chemical Industries, Ltd., Japan) and 0.15 M NaCl, pH 7.2].

A conjugate (80 µg/mℓ) prepared by combining a Fab fragment of rabbit anti-human immunoglobulin antibody [Eiji Ishikawa, et al.: Enzyme Immunoassay, published by IGAKU SHOIN LTD. (1978)] with horseradish peroxidase (a product of Sigma Co.) by the periodic acid method (the reference mentioned above) was diluted 20-fold with buffer solution B and 100 µℓ of the thus obtained solution was placed in each well. After being allowed to stand at room temperature for 2 hours, the wells were washed three times with 150 µℓ each of buffer solution B. Then, 100 µℓ of an orthophenylenediamine substrate solution (a 1/15 M sodium phosphate buffer solution containing 0.02% orthophenylenediamine and 35 mM hydrogen peroxide, pH 7.2) was added to each well, and the plates were allowed to stand at room temperature for 15 minutes. The reaction was discontinued by adding 50 µℓ of 2 N $H_2SO_4$ to each well, and the plates were allowed to stand at room temperature for 10 minutes. Absorbancies (O.D.) at 410 nm and 600 nm were measured with a photometer for microtiter plate, Model MTP-22 (a product of Corona Co.) and antibody values were obtained as differences between the absorbancies. Tests were all carried out in two series, and averages of the two measurements were employed.

(2) The same samples as used in the foregoing section (1) were subjected to anti-ATLV antibody detection according to the procedure disclosed in Rinsho Kensa (Clinical Inspection) 29 (1): 91-94 (1985) (Eisai method) using Eitest® ATL (a product of Eisai Co., Ltd., Japan).

(3) Averages and standard deviation values of measurements (O.D.) of serums of 30 normal men carried out in the foregoing sections (1) and (2) are as follows:

gag-env 0.055 ± 0.013

p24 0.010 ± 0.004

Eisai 0.040 ± 0.009

Cut-off value (the sum of the average and double the standard deviation) for p24 is 0.018, that for the gag-env protein 0.081 and that for Eisai 0.059.

The results of measurements of ATL patients and HTLV-1 carriers (a total of 57 individuals) are shown in Table 1.

Table 1

| Sample No. | Antibody value | | | Sample No. | Antibody value | | |
|---|---|---|---|---|---|---|---|
| | gag-env | p24 | Eisai | | gag-env | p24 | Eisai |
| 1 | .299 | .042 | .696 | 30 | .580 | .442 | 1.609 |
| 2 | .660 | .390 | 1.531 | 31 | .463 | .338 | 1.018 |
| 3 | .401 | .055 | .754 | 32 | .608 | .315 | 1.184 |
| 4 | .519 | .276 | 1.214 | 33 | .637 | .291 | .748 |
| 5 | .423 | .069 | .762 | 34 | .423 | .187 | .254 |
| 6 | .258 | .035 | .604 | 35 | .593 | .288 | .480 |
| 7 | .188 | .009 | .248 | 36 | .249 | .106 | .818 |
| 8 | .139 | .009 | .161 | 37 | .423 | .214 | .936 |
| 9 | .666 | .100 | .615 | 38 | .259 | .072 | .780 |
| 10 | .111 | .004 | .073 | 39 | .340 | .234 | .806 |
| 11 | .232 | .012 | .150 | 40 | .362 | .184 | .734 |
| 12 | .443 | .147 | .499 | 41 | .468 | .247 | .322 |
| 13 | .386 | .172 | .208 | 42 | .323 | .094 | .460 |
| 14 | .235 | .023 | .431 | 43 | .224 | .074 | .392 |
| 15 | .089 | .008 | .140 | 44 | .192 | .020 | .133 |
| 16 | .555 | .293 | 1.205 | 45 | .347 | .065 | .426 |
| 17 | .341 | .042 | .600 | 46 | .596 | .269 | .865 |
| 18 | .329 | .023 | .262 | 47 | .303 | .061 | .312 |
| 19 | .408 | .278 | .741 | 48 | .390 | .105 | .595 |
| 20 | .495 | .074 | .785 | 49 | .572 | .393 | 1.591 |
| 21 | .406 | .115 | .729 | 50 | .299 | .040 | .393 |
| 22 | .338 | .054 | .116 | 51 | .217 | .021 | .223 |
| 23 | .305 | .204 | .907 | 52 | .304 | .043 | .163 |
| 24 | .488 | .256 | 1.207 | 53 | .422 | .036 | .313 |
| 25 | .108 | .011 | .190 | 54 | .284 | .123 | .529 |
| 26 | .175 | .078 | .204 | 55 | .096 | .009 | .041 |
| 27 | .162 | .015 | .122 | 56 | .112 | .007 | .079 |
| 28 | .147 | .010 | .074 | 57 | .605 | .424 | 1.340 |
| 29 | .379 | .160 | .524 | | | | |

Table 2 shows the percentage of positive cases calculated from the results in Table 1 and the cut-off values.

Table 2

| Antigen | Percentage of positive cases | |
|---|---|---|
| | 30 normal men | ATL patients and HTLV-1 carriers (a total of 57 individuals) |
| gag-env | 0/30 (0%) | 57/57 (100%) |
| p24 | 0/30 (0%) | 47/57 (82.5%) |
| Eisai | 0/30 (0%) | 56/57 (98.2%) |

It can be seen from Table 2 that the percentage of the false positive cases is 0% with normal men in all the procedures and that 10 cases among the ATL patients and HTLV-1 carriers are false negative (less than cut-off value 0.018) in the p24 method and one case is false negative (less than cut-off value 0.059) in the Eisai method, whereas there is no false negative case (less than cut-off value 0.081) in the gag-env protein method (percentage of positive cases: 100%).

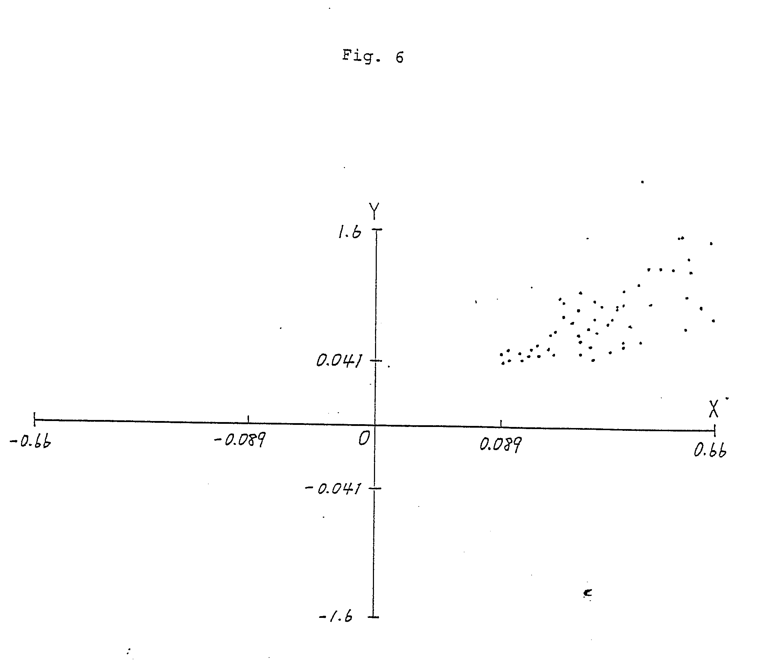

Correlations between the result of the Eisai method and that of the p24 method or that of the gag-env method are shown in Figs. 5 and 6. Correlation coefficient was 0.8294 between the result of the Eisai method and that of the p24 method and 0.7479 between the result of the Eisai method and that of the gag-env method. The values of the correlation coefficient show that the results of the Eisai method, the p24 method and the gag-env method have correlation with one another.

Reference Example 1

(1) Isolation and purification of plasmids pKYP26 and pEFM2:

Escherichia coli IKYP26 (FERM BP-863) carrying pKYP26 and Escherichia coli EEFM2 (ATCC 53228) carrying pEFM2 were respectively cultured in 10 mℓ of L medium (1% Bacto-Tryptone, 0.5% yeast extract, 1% NaCl, pH 7.5) containing 50 ug/mℓ ampicillin at 37°C for 18 hours. The whole culture broth was inoculated into 1 ℓ of L medium containing 50 µg/mℓ ampicillin and cultured at 37°C. After 4 hours, 170 µg/mℓ chloramphenicol was added and culturing was continued at 37°C for 16 hours. Cells were collected by centrifugation (5,000 rpm, 10 minutes) and washed with 0.8% NaCl. The washed cells were suspended in 20 mℓ of 50 mM Tris-HCl (pH 8.0) and cooled with ice. 8 mℓ of 10 mg/mℓ lysozyme was added and the mixture was allowed to stand under ice cooling for 10 minutes. 9.6 mℓ of 0.5 M EDTA was added and the mixture was allowed to stand under ice cooling for 10 minutes. 2.3 mℓ of 2% Triton X-100 (a product of Wako Pure Chemical Industries, Ltd.) was added and the mixture was allowed to stand under ice cooling for one hour. The mixture was subjected to ultracentrifugation at 50,000 × g at 4°C for one hour to obtain about 40 mℓ of a supernatant. The pH of the supernatant was adjusted to 12.5 with 3 M NaOH and the supernatant was stirred gently at room temperature for 10 minutes. After the pH was readjusted to 8.5 with 2 M Tris-HCl (pH 7.5), the solution was stirred for 3 minutes. The volume of the solution was about 55 mℓ at this point. 5 M NaCl (1/9 of the volume of the solution) and phenol saturated with 10 mM Tris-HCl (pH 7.5) and 1 mM EDTA (equal to the volume of the solution) were added. The mixture was vigorously stirred and subjected to low pressure centrifugation (3,300 rpm, 10 minutes, the same conditions will be employed hereinafter) to recover a water layer (this treatment is referred to as "phenol extraction" hereinafter). 5 mg/mℓ RNase A (a product of Sigma Co.) (1/250 of the volume of the water layer) was added and RNA decomposition reaction was carried out at 37°C for one hour. Then, 5 M NaCl (1/5 of the volume of the

reaction mixture) and 30% PEG 6000 (a product of Nakarai Chemicals, Ltd.) (1/3 of the volume of the reaction mixture) were added. The mixture was allowed to stand at -20°C for 2 hours. A precipitate recovered by centrifugation was dissolved in 2 mℓ of a solution comprising 10 mM Tris-HCl (pH 7.5) and 1 mM EDTA. 0.5% sodium dodecyl sulfate (SDS) was added and 50 μg/mℓ Proteinase K (a product of Sigma Co.) was added. Protein decomposition reaction was carried out at 37°C for one hour. Phenol extraction was repeated three times and an equal amount of chloroform was added. The mixture was vigorously stirred and subjected to low pressure centrifugation to recover a water layer. 3 M sodium acetate (1/10 of the volume of the water layer) and ethanol (2.5 times the volume of the water layer) were added. The mixture was allowed to stand at -20°C for one hour. A precipitate was recovered by centrifugation with cooling (4°C, 11,000 rpm, 10 minutes) and dissolved in 1 mℓ of a solution comprising 10 mM Tris-HCl (pH 7.5) and 1 mM EDTA. Thus, 800 μg each of pKYP26 and pEFM2 were obtained. The structure of pKYP26 was confirmed by cleavage with EcoRI, KpnI, BamHI, BglII and PstI and agarose gel electrophoresis. The structure of pEFM2 was confirmed by cleavage with XhoI, KpnI, BglII, HpaI and PstI and agarose gel electrophoresis.

(2) Construction of a plasmid DNA containing p24 gene of ATLV:

(2)-1 Construction of pAFB7:

5 μg of pGEL1 obtained from Escherichia coli IGEL1 (FERM BP-629) by a conventional method was dissolved in 40 μℓ of a solution comprising 10 mM Tris-HCl (pH 7.5), 7 mM $MgCl_2$, 6 mM mercaptoethanol and 100 mM NaCl (referred to as Y-100 buffer solution hereinafter). 10 units of restriction enzyme PstI (a product of Takara Shuzo Co., the restriction enzymes used hereinafter are all products of Takara Shuzo Co., unless otherwise specified) and 8 units of BamHI were added and digestion reaction was carried out at 37°C for 2 hours. About 0.2 μg of a DNA fragment of about 1,700 bp containing lpp terminator was recovered from the reaction solution by low gelling temperature agarose gel electrophoresis [L. Wieslander: Analytical Biochemistry 98 , 305 (1979), referred to as LGT method hereinafter].

Separately, 10 μg of pGEL1 was dissolved in 100 μℓ of Y-100 buffer solution and 16 units of restriction enzyme PstI was added. Digestion reaction was carried out at 37°C for one hour. After confirmation of the digestion with PstI by agarose gel electrophoresis, 3 units of restriction enzyme HpaI was added and partial digestion reaction with HpaI was carried out at 37°C for 30 minutes. About 0.2 μg of a partially digested DNA fragment of about 940 bp containing trp promoter was obtained from the reaction solution by the LGT method.

Then, 30 μg of pAFA10 which is the plasmid carried on ATCC 39582 strain and illustrated in Fig. 1 was dissolved in 250 μℓ of Y-100 buffer solution. 140 units of restriction enzyme HpaI and 30 units of BamHI were added and digestion reaction was carried out at 37°C for 3 hours. About 0.5 μg of a DNA fragment of about 700 bp containing p24 gene was obtained from the reaction solution by the LGT method.

About 0.1 μg of the PstI-BamHI fragment (about 1,700 bp) derived from pGEL1, about 0.15 μg of the PstI-HpaI fragment (about 940 bp) derived from pGEL1 and about 0.2 μg of the HpaI-BamHI fragment (about 700 bp) derived from pAFA10 were dissolved in 20 uℓ of a solution comprising 20 mM Tris-HCl (pH 7.5), 10 mM $MgCl_2$, 10 mM dithiothreitol and 1 mM ATP (referred to as T4DNA ligase buffer hereinafter). 3 units of T4DNA ligase (a product of Takara Shuzo Co.) was added and ligation reaction was carried out at 4°C for 16 hours.

Escherichia coli HB101 [Bolivar, etal., Gene 2, 75 (1977)] was transformed using the reaction solution to obtain $Ap^R$ colonies. Plasmid DNA, pAFB7 illustrated in Fig. 1 was obtained from one of the colonies by the method of Birnboim, et al. [Nucleic Acids Research 7, 1513 (1979)].

(2)-2 Construction of pAAB6:

2 μg of pTAG424A which is the plasmid carried on FERM BP-341 strain and illustrated in Fig. 2 (JP-A-61534/85) was dissolved in 20 μℓ of Y-100 buffer solution. 4 units of restriction enzyme BamHI was added and digestion reaction was carried out at 37°C for 2 hours. Then, the NaCl concentration of the solution was adjusted to 150 mM and 4 units of restriction enzyme NcoI (a product of Nippon Gene Co.) was added. Digestion reaction was carried out at 37°C for 2 hours. The BamHI-NcoI cleaved DNA was dissolved in 20 μℓ of a solution comprising 33 mM Tris-acetic acid (pH 7.9), 66 mM potassium acetate, 10 mM magnesium acetate, 5 mM dithiothreitol and 0.4 mM each dATP, dCTP, dGTP and dTTP (referred to as T4DNA

polymerase buffer hereinafter). One unit of T4DNA polymerase (a product of Takara Shuzo Co.) was added and reaction was carried out at 37°C for 30 minutes.

The enzyme-treated solution was mixed with 20 μl of T4DNA ligase buffer and 3 units of T4DNA ligase was added. Ligation reaction was carried out at 4°C for 18 hours.

Escherichia coli HB101 was transformed using the reaction solution to obtain $Ap^R$ colonies. Plasmid DNA, pAAB6 illustrated in Fig. 2 was recovered from one of the colonies by the method of Birnboim, et al.

(2)-3 Construction of pAHA1:

15 μg of pAAB6 obtained above was dissolved in 100 μl of Y-100 buffer solution and 15 units of restriction enzyme StuI and 20 units of ClaI (a product of New England Bio Labs) were added. Digestion reaction was carried out at 37°C for 3 hours. About 0.3 μg of a DNA fragment of about 400 bp containing the first half of p24 gene was obtained from the reaction solution by the LGT method.

Then, 5 μg of pGEL1 was dissolved in 40 μl of Y-100 buffer solution and 5 units of restriction enzyme ClaI (a product of New England Bio Labs) and 8 units of PstI were added. Digestion reaction was carried out at 37°C for 2 hours. About 0.2 μg of a DNA fragment of about 1,000 bp containing trp promoter was obtained from the reaction solution by the LGT method.

Separately, 2 μg of pKYP26 obtained in (1) above was dissolved in 30 μl of a solution comprising 10 mM Tris-HCl (pH 7.5), 7 mM $MgCl_2$, 6 mM mercaptoethanol and 10 mM NaCl (referred to as Y-10 buffer solution hereinafter). 5 units of restriction enzyme KpnI was added and digestion reaction was carried out at 37°C for 2 hours. The KpnI cleaved DNA was dissolved in 20 μl of T4DNA polymerase buffer and one unit of T4DNA polymerase was added. Reaction was carried out at 37°C for 30 minutes. The DNA reaction solution was dissolved in 30 μl of Y-100 buffer solution and 4 units of restriction enzyme PstI was added. Digestion reaction was carried out at 37°C for 2 hours. About 0.2 μg of a DNA fragment of about 1,700 bp containing lpp terminator was obtained from the reaction solution by the LGT method.

About 0.1 μg of the StuI-ClaI fragment (about 400 bp) derived from pAAB6, about 0.2 μg of the ClaI-PstI fragment (about 1,000 bp) derived from pGEL1 and about 0.1 μg of the KpnI-PstI fragment (about 1,700 bp) derived from pKYP26 were dissolved in 30 μl of T4DNA ligase buffer. 4 units of T4DNA ligase was added and ligation reaction was carried out at 4°C for 18 hours.

Escherichia coli HB101 was transformed using the reaction solution to obtain $Ap^R$ colonies. Plasmid DNA, pAHA1 illustrated in Fig. 2 was recovered from one of the colonies by the method of Birnboim, et al.

(2)-4 Construction of pAFG10:

5 μg of pAHA1 obtained above was dissolved in 50 μl of Y-100 buffer solution and 8 units of restriction enzyme PstI was added. Digestion reaction was carried out at 37°C for 2 hours. About 0.5 μg of a DNA fragment of about 1,100 bp containing trp promoter and an N-terminal part of p24 gene was obtained from the reaction solution by the LGT method.

Then, 6 μg of pAFB7 constructed in (2)-1 above was dissolved in 30 μl of Y-100 buffer solution. 8 units of restriction enzyme BamHI was added and digestion reaction was carried out at 37°C for 2 hours. 5 μl of a solution comprising 0.2 M Tris-HCl (pH 8.0), 120 mM $CaCl_2$, 120 mM $MgCl_2$, 2 M NaCl and 10 mM EDTA was added to the reaction solution. 15 μl of sterilized water was added and the solution was kept at 30°C for 3 minutes. 10 units of BAL31 nuclease (a product of Bethesda Research Laboratories) was added and decomposition reaction was carried out at 30°C for 80 seconds. After reaction, phenol extraction was carried out and a DNA was recovered by ethanol precipitation. The DNA was dissolved in 30 μl of Y-100 buffer solution and 8 units of restriction enzyme PstI was added. Digestion reaction was carried out at 37°C for 2 hours. About 0.1 μg of a DNA fragment of about 450 bp containing p24 gene was obtained from the reaction solution by the LGT method.

About 0.2 μg of the PstI fragment (about 1,100 bp) derived from pAHA1, 0.1 μg of the PstI-BAL31 nuclease decomposition fragment (about 450 bp) derived from pAFB7 and 0.1 μg of the KpnI-PstI fragment (about 1,700 bp) derived from pKYP26 were dissolved in 30 μl of T4DNA ligase buffer. 6 units of T4DNA ligase was added and ligation reaction was carried out at 4°C for 18 hours.

Escherichia coli HB101 was transformed using the reaction solution to obtain $Ap^R$ colonies. Plasmid DNA, pAFG10 illustrated in Fig. 3 was recovered from one of the colonies by the method of Birnboim, et al. The base sequence of the C-terminal region coding for p24 was determined and it was confirmed that the region contained the C-terminal of p24 and Val•Val•Leu•Ser•Asn was attached thereto.

(3) Construction of recombinant plasmid pETI7 coding for a hybrid antigen polypeptide wherein an antigen polypeptide encoded by the gag gene of ATLV and an antigen polypeptide encoded by the env gene of ATLV are fused:

10 μg of recombinant plasmid pAFG10 obtained in (2)-4 above was dissolved in 100 μℓ of Y-100 buffer solution. 10 units of restriction enzyme XhoI and 12 units of StuI were added and digestion reaction was carried out at 37°C for 3 hours.

About 0.5 μg of a DNA fragment of about 630 bp containing trp promoter and the first half of p24 gene was obtained from the reaction solution by the LGT method.

Then, 5 μg of pEFM2 obtained in (1) above was dissolved in 50 μℓ of Y-100 buffer solution and 10 units of restriction enzyme XhoI and 8 units of HpaI were added. Digestion reaction was carried out at 37°C for 3 hours. About 1 μg of a DNA fragment of about 2,700 bp containing the second half of env gene was obtained from the reaction solution by the LGT method.

0.05 pmole of the XhoI-StuI fragment (about 630 bp) derived from pAFG10 and 0.01 pmole of the XhoI-HpaI fragment (about 2,700 bp) derived from pEFM2 were dissolved in 40 μℓ of T4DNA ligase buffer. 5 units of T4DNA ligase was added and ligation reaction was carried out at 4°C for 18 hours.

Escherichia coli HB101 was transformed using the reaction solution to obtain $Ap^R$ colonies. Plasmid DNA, pETI7 illustrated in Fig. 4 was recovered from one of the colonies by the method of Birnboim, et al.

(4) Production in Escherichia coli carrying pETI7 of the fused polypeptide wherein an antigen polypeptide encoded by the gag gene and an antigen polypeptide encoded by the env gene are fuse:

Escherichia coli W3110StrA (FERM BP-732) was transformed by a conventional method using recombinant plasmid pETI7 obtained in (3) above. An $Ap^R$ colony was selected and inoculated into 8 mℓ of MCG medium [0.6% $Na_2HPO_4$, 0.3% $KH_2PO_4$, 0.5% NaCl, 0.1% $NH_4Cl$, 0.5% glucose, 0.5% Casamino acid, 1 mM $MgSO_4$, 4 μg/mℓ vitamine $B_1$, pH 7.2]. Culturing was carried out at 30°C for 18 hours. The culture broth was centrifuged at 8,000 rpm for 10 minutes to recover cells. The cells were suspended in Sample buffer of Laemmli. The suspension was subjected to SDS-polyacrylamide gel electrophoresis and staining with Coomassie Brilliant Blue to detect a polypeptide band of a molecular weight of about 25,000. The band was not detected in the case of Escherichia coli which does not carry the plasmid.

The molecular weight of the polypeptide coincided with the molecular weight of the hybrid antigen polypeptide (25,051.02) calculated from the structure of plasmid pETI7.

(5) The cells obtained from 1 ℓ of the culture broth mentioned in (4) above by centrifugation (8,000 rpm, 30 minutes) were suspended in 200 mℓ of deionized water and disrupted under a pressure of 400 kg/cm$^2$ in a Manton-Gaulin homogenizer (Manton-Gaulin Manufacturing Co., Inc. USA), and a precipitate fraction was obtained therefrom by centrifugation. It was found by SDS-polyacrylamide electrophoresis that the precipitate fraction contained 845 mg of the gag-env protein.

The precipitate fraction was dispersed in 20 mM phosphate buffer (pH 7.4) containing 4% (W/V) Triton X-100 and 10 mM sodium ethylenediaminetetraacetate, and a precipitate fraction was again obtained by centrifugation. The precipitate was dissolved in a phosphate buffer solution (pH 7.4) containing 2 mM sodium ethylenediaminetetraacetate, 0.3% (V/V) 2-mercaptoethanol and 7 M urea, and the solution was passed through a column containing 100 mℓ of TSK gel CM Toyopearl 650 (a product of Toyo Soda Manufacturing Co., Ltd., Japan) equilibrated in advance with 20 mM phosphate buffer solution containing 7 M urea (hereinafter referred to as equilibrating buffer solution, pH 7.0) to effect adsorption.

The column was washed with the equilibrating buffer solution and eluted with the equilibrating buffer solution containing 0.1 M NaCl to obtain 250 mℓ of a fraction of high purity. The fraction, which contained 430 mg of the gag-env protein, was dialyzed against 3 ℓ of 10 mM carbonate buffer solution (pH 9.4) as an external liquid. The resulting precipitates were removed by centrifugation, and 400 mℓ of a solution of the desired gag-env protein was obtained as a supernatant. It was found by SDS-PAGE that the protein purity was 90% or more and the yield was 160 mg.

## Claims

1. A method for detecting an anti-adult T cell leukemia virus antibody, characterized by detecting the anti-adult T cell leukemia virus antibody in a sample according to an immunological procedure using a polypeptide encoded by a fused gene comprising the gag gene of an adult T cell leukemia virus (hereinafter referred to as ATLV) or a part thereof and the env gene of ATLV or a part thereof as an antigenic substance.

2. A method according to Claim 1, wherein the immunological procedure is a radio immunoassay, enzyme immunoassay, fluorescence immunoassay, reversed passive agglutination, phytohematoagglutination, particle agglutination, laser nephrometry or an immune hemolysis procedure using erythrocytes or liposomes.

3. A method according to Claim 1, wherein the antigenic substance is used in the form of a coating on a solid phase.

4. A method according to Claim 3, wherein said solid phase is a tube made of synthetic resin or glass, glass beads or a microtiter plate made of glass.

FIG. 1

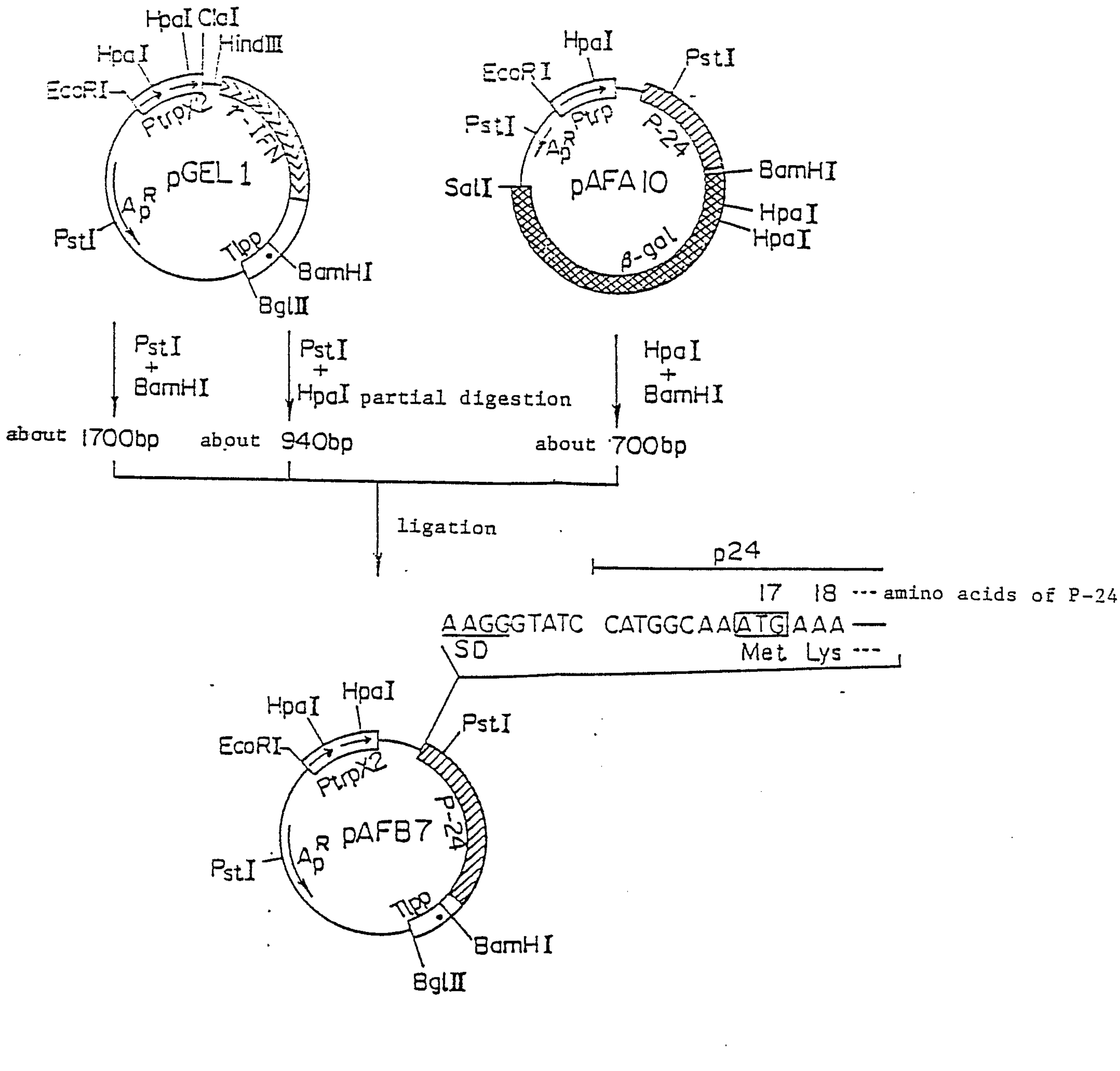


BAD ORIGINAL

FIG. 2

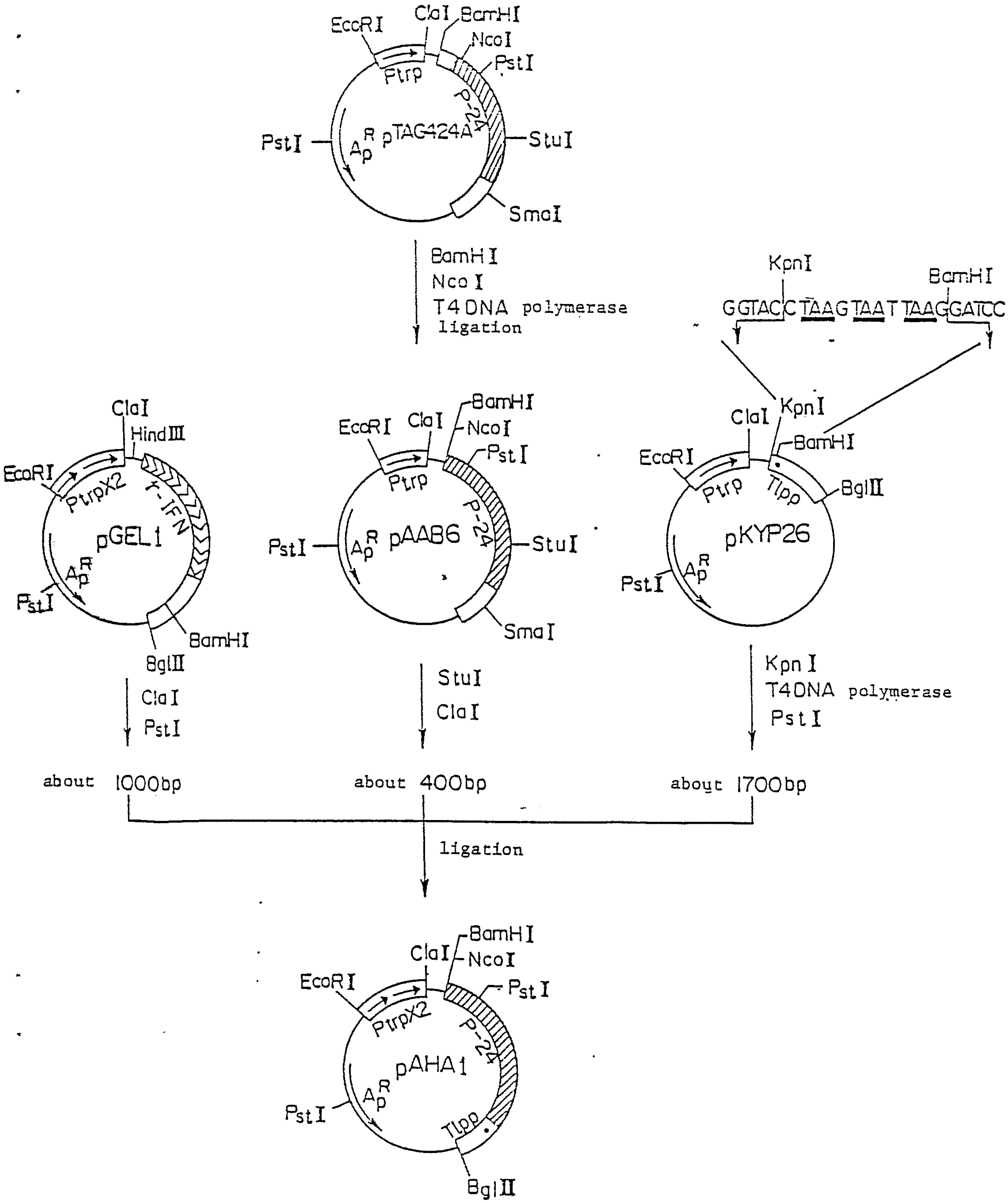
EcoRI
ClaI
BamHI
NcoI
PstI
Ptrp
P-24
pTAG424A
$A_p^R$
StuI
SmaI
BamHI
NcoI
T4DNA polymerase
ligation
KpnI
BamHI
GGTACCTAAGTAATTAAGGATCC
ClaI
HindIII
PtrpX2
γ-IFN
pGEL1
BglII
pAA86
pKYP26
Tlpp
BglII
ClaI
PstI
StuI
ClaI
KpnI
T4DNA polymerase
PstI
about 1000bp
about 400bp
about 1700bp
ligation
pAHA1
BglII

FIG. 3

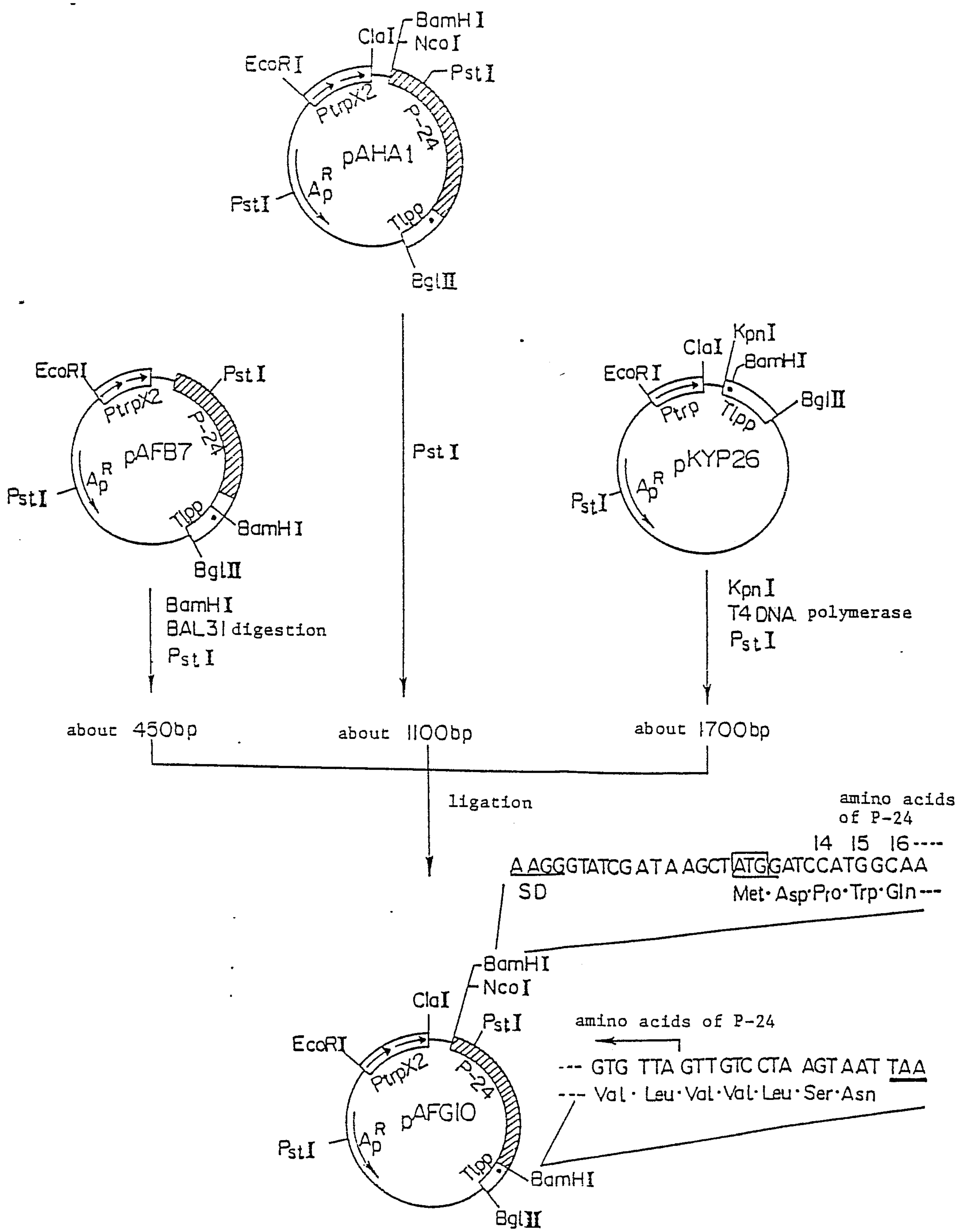

BamHI
ClaI
NcoI
EcoRI
PstI
PtrpX2
P-24
pAHA1
Ap R
PstI
Tlpp
BglII
EcoRI
PstI
PtrpX2
P-24
pAFB7
Ap R
PstI
Tlpp
BamHI
BglII
PstI
KpnI
ClaI
BamHI
EcoRI
BglII
Ptrp
Tlpp
pKYP26
Ap R
PstI
BamHI
BAL31 digestion
PstI
KpnI
T4 DNA polymerase
PstI
about 450bp
about 1100bp
about 1700bp
ligation
amino acids of P-24
14 15 16 ----
AAGGGTATCGATAAGCTATGGATCCATGGCAA
SD
Met·Asp·Pro·Trp·Gln---
BamHI
NcoI
ClaI
PstI
EcoRI
PtrpX2
P-24
pAFG10
Ap R
PstI
Tlpp
BamHI
BglII
amino acids of P-24
--- GTG TTA GTT GTC CTA AGT AAT TAA
--- Val · Leu · Val · Val · Leu · Ser · Asn

FIG. 4

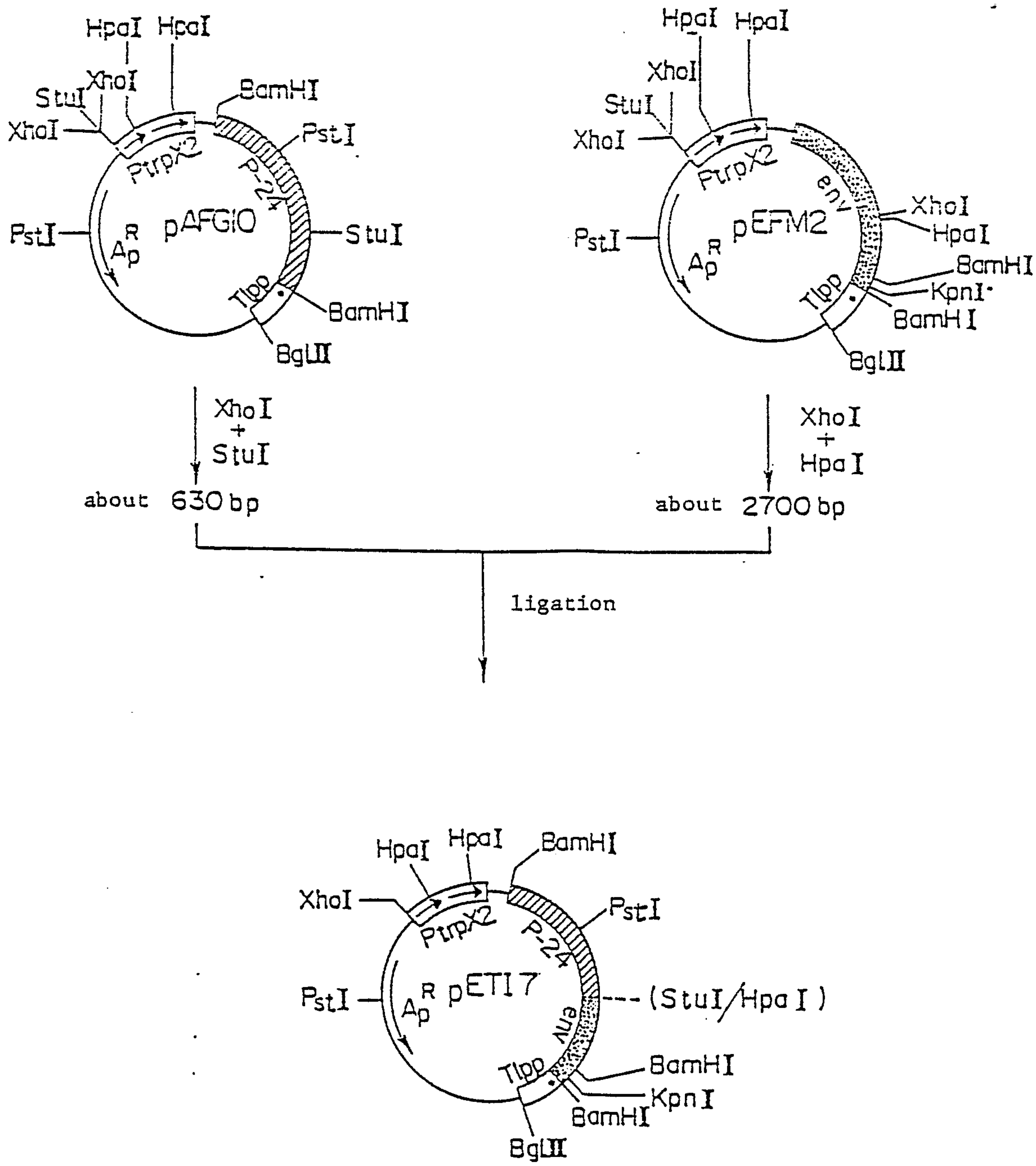
HpaI HpaI
XhoI
StuI
XhoI
BamHI
PstI
PtrpX2
P-24
pAFG10
PstI
StuI
$A_p^R$
Tlpp
BamHI
BglII
HpaI HpaI
XhoI
StuI
XhoI
PtrpX2
env
XhoI
HpaI
pEFM2
PstI
$A_p^R$
BamHI
KpnI
Tlpp
BamHI
BglII
XhoI + StuI
about 630 bp
XhoI + HpaI
about 2700 bp
ligation
HpaI HpaI BamHI
XhoI
PtrpX2
P-24
PstI
pET17
PstI
$A_p^R$
(StuI/HpaI)
env
Tlpp
BamHI
KpnI
BamHI
BglII

Fig. 5

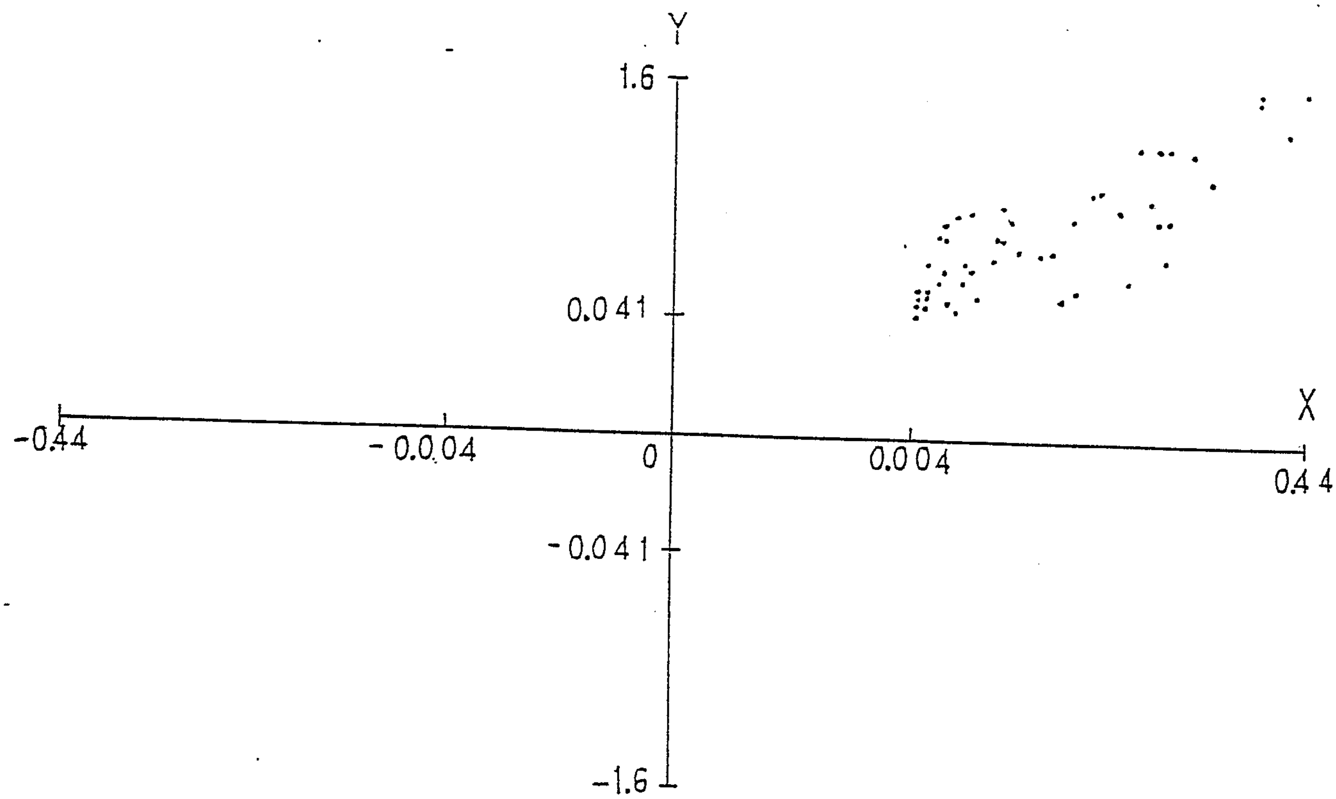

Fig. 6